# FASCICULE DE BREVET EUROPEEN

(11) **EP 0 653 194 B1**
(45) Date de publication et mention de la délivrance du brevet: **17.03.1999**
(21) Numéro de dépôt: 93420451.2
(22) Date de dépôt: 12.11.1993
(51) Int. Cl.: A61F 2/38

(54) **Prothèse totale du genou**
Ganzknieprothese
Total knee prosthesis

(43) Date de publication de la demande: 17.05.1995
(73) Titulaire: FABRIQUE D'IMPLANTS ET D'INSTRUMENTS CHIRURGICAUX SOCIETE A RESPONSABILITE LIMITEE, F-43240 Saint Just Malmont (FR)
(72) Inventeur: Cuilleron Jean, 42100 SAINT ETIENNE (FR)
(74) Mandataire: Dupuis, François

(56) Documents cités:
- EP-A- 0 177 755
- EP-A- 0 265 325
- EP-A- 0 472 475
- WO-A-79/00739
- DE-A- 2 114 287
- FR-A- 2 290 883
- FR-A- 2 601 873
- GB-A- 1 328 497

## Description

On rappelle de manière parfaitement connue pour un homme du métier que ce type de prothèse comprend un élément fémoral et un élément tibial accouplés entre eux de manière articulée, pour permettre des mouvements de flexion.

Par exemple, on peut citer des prothèses dont l'élément fémoral est en appui par des patins condyliens sur une embase tibiale. L'élément fémoral est accouplé à l'élément tibial au moyen d'une tige verticale engagée à libre coulissement dans une partie complémentaire que présente ledit élément tibial. Ces dispositions permettent d'obtenir des mouvements combinés de flexion et de rotation. Des moyens limitent et contrôlent la rotation entre l'élément fémoral et l'élément tibial. De même, des agencements peuvent empêcher tout mouvement de déplacement relatif entre l'élément fémoral et l'élément tibial après accouplement, pour éviter tout problème de luxation. Les caractéristiques d'une telle prothèse ressortent par exemple de l'enseignement du brevet FR 2.601.873.

On connaît également des prothèses du genou dites à glissement, c'est-à-dire où l'élément fémoral et tibial sont indépendants. Dans ce type de prothèse, on a proposé d'intercaler entre l'élément fémoral et l'élément tibial, un élément intermédiaire parfois appelé élément méniscal. Cet élément intermédiaire peut être totalement indépendant des éléments fémoral et tibial ou être lié à l'élément tibial, notamment avec l'embase tibiale, avec capacité de déplacement angulaire limité et contrôlé.

Ces dispositions permettent d'augmenter les surfaces en contact des éléments fémoral et tibial, en diminuant en conséquent l'usure, tout en améliorant le guidage.

Cet état de la technique ressort par exemple de l'enseignement du brevet FR 2.663.536.

On peut citer également la demande de brevet EP 0472475 concernant une prothèse bi-condylienne du genou. Cette prothèse comprend un élément fémoral et un élément tibial articulé l'un à l'autre par un axe d'articulation. L'axe d'articulation est monté dans un support présentant une tige de section oblongue engagée dans un put ménagé dans le plateau tibial. Le plateau tibial présente des ergots coopérant avec des lumières formées dans l'épaisseur de l'embase tibiale.

Il apparait que la tige ne participe pas au guidage latéral par rapport à l'embase tibiale. Tous les efforts latéraux sont transmis à l'embase tibiale par le plateau en polyéthylène. En outre, le mouvement de flexion en extension n'est limité que par le contact des patins condyliens dans des saignées formées dans l'épaisseur du plateau tibial. En position de butée, les patins condyliens peuvent grimper sur l'extrémité du plateau, et créer un récurvatum du fémur, par rapport au tibia, en provoquant ainsi un déboitage de la tige verticale.

Le problème que se propose de résoudre l'invention est de réaliser une prothèse totale du genou, c'est-à-dire une prothèse où l'élément fémoral et tibial sont accouplés à libre articulation, combinant les avantages des deux techniques décrites ci-dessus.

Pour résoudre un tel problème, la prothèse selon l'invention est telle que définie dans les revendications et comprend en combinaison :
- un élément fémoral recevant un axe transversal d'articulation monté à libre rotation par rapport à une tige cylindrique engagée dans un fourreau d'un élément tibial pour permettre en combinaison des mouvements de flexion et de rotation ;
- un élément intermédiaire sous forme d'un plateau en polyéthylène monté avec capacité de déplacement angulaire limité dans un plan horizontal sur l'embase tibiale, ledit élément recevant en appui, l'élément fémoral avec capacité de flexion angulaire.

Pour résoudre le problème posé d'assurer l'articulation entre l'élément fémoral et l'élément tibial, l'axe d'articulation est monté libre en rotation dans un manchon transversal solidaire du fourreau vertical de l'élément tibial, ledit manchon étant engagé dans une saillie creuse de l'élément fémoral, constituant une chape.

Pour résoudre le problème posé d'obtenir une articulation d'un fonctionnement sûr et efficace, l'axe d'articulation est monté dans le manchon au moyen d'organes de roulement.

Avantageusement, les organes de roulement sont du type à aiguille.

Un autre problème que se propose de résoudre l'invention est de limiter la flexion entre l'élément tibial et l'élément fémoral.

Un tel problème est résolu en ce que le manchon présente très sensiblement au niveau du plateau tibial, un bec faisant office de butée, coopérant avec une partie de la salle, en position de flexion de l'élément fémoral.

Pour résoudre le problème posé de tenir compte du mouvement de rotation naturel entre le fémur et le tibia au moment de la flexion, le plateau est solidaire de l'élément tibial avec capacité de déplacement angulaire, au moyen de pions solidaires dudit élément et coopérant par une tête profilée avec des lumières profilées, formées dans l'épaisseur dudit plateau.

Un autre problème que se propose de résoudre l'invention est d'assurer l'accouplement et le désaccouplement des éléments tibial et fémoral sans être obligé d'intervenir sur des organes rapportés, en ayant pour objectif de pouvoir accoupler les deux éléments sous un faible effort de pression et de les désaccoupler seulement sous un effort de traction plus important.

Pour résoudre un tel problème, le fourreau de l'élément tibial recevant la tige cylindrique de l'élément fémoral présente au moins un moyen apte à coopérer en pression avec ladite tige, ledit moyen étant conformé pour être sollicité élastiquement en combinaison avec des agencements de la tige et du fourreau, pour permettre :
- l'introduction de la tige dans l'aléasage selon une force d'intensité réduite et de blocage en position,
- le retrait de la tige de l'alésage selon une force d'intensité supérieure à la précédente, correspondant au désaccouplement.

L'invention est exposée, ci-après plus en détail à l'aide des dessins annexés, dans lesquels :

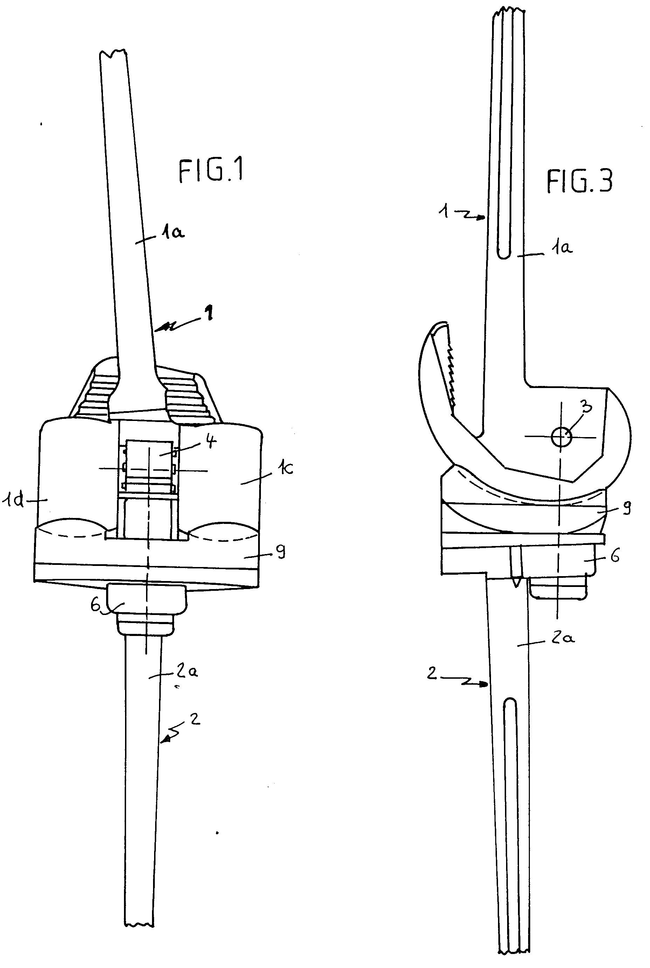

La figure 1 est une vue de face de la prothèse considérée du côté postérieur.
La figure 2 est une vue de face correspondant à la figure 1 considérée du côté antérieur.
La figure 3 est une vue de profil correspondant à la figure 1.
La figure 4 est une vue partielle en coupe longitudinale considérée au niveau de l'articulation de l'élément tibial et de l'élément fémoral. On a illustré en traits interrompus l'élément fémoral en position de flexion.
La figure 5 est une vue en coupe partielle de la figure 4.
La figure 6 est une vue en coupe de la figure 4.

On rappelle pour une meilleure compréhension de la suite de la description, que la prothèse comprend de manière connue un élément fémoral (1) et un élément tibial (2). Chacun des éléments (1) et (2) présente directement ou de manière rapportée, une tige d'ancrage (1a) et (2a) pour leur fixation, respectivement dans le canal médullaire du fémur et du tibia.

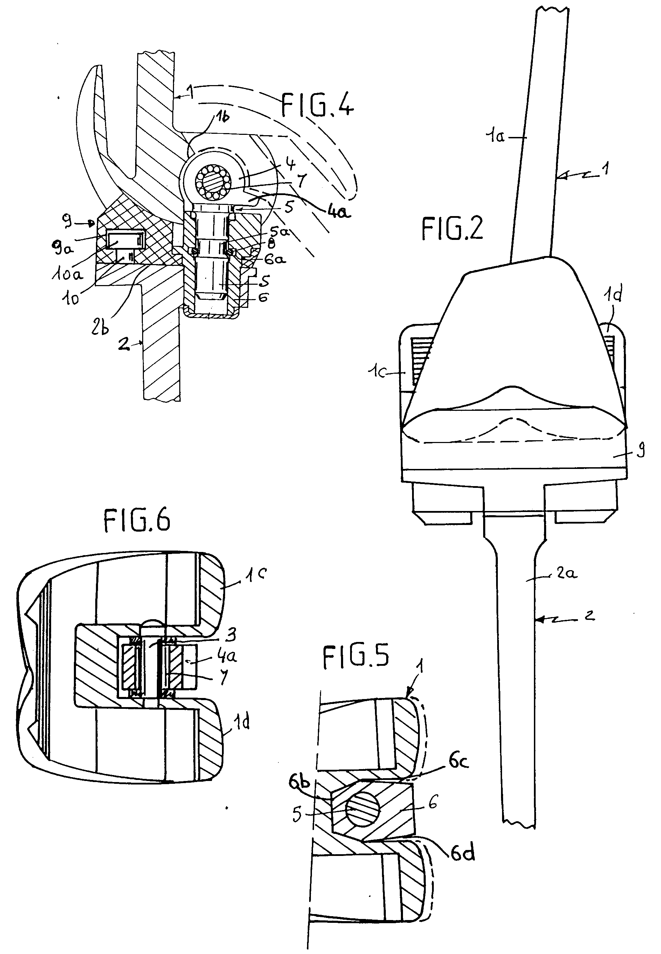

L'élément fémoral (1) reçoit un axe transversal (3) monté à libre rotation dans un manchon (4) solidaire d'une tige cylindrique (5) engagée librement dans un fourreau vertical (6) que présente l'élément tibial (2). Le manchon (4) est engagé dans une salle creuse (1b) formée dans la partie médiane de l'élément fémoral (1) , entre les patins condyliens (1c) et (1d). Cette échancrure (1b) constitue une chape. L'axe (3) est monté dans le manchon (4), en combinaison avec des organes de roulement (7) sous forme notamment, de roulement à aiguilles (figure 4).

Le manchon (4) présente très sensiblement au niveau du fourreau (6), un bec (4a) faisant office de butée en position de flexion de l'élément fémoral. Ce bec (4a) reçoit en butée, une partie (1b1) de la saillie (1b) de l'élément fémoral (1) (tracé traits interrompus) (figure 4).

Ces dispositions permettent donc un mouvement de flexion de l'élément fémoral (1) par rapport à l'élément tibial (2), compte-tenu du montage de l'axe (3). De même, la tige cylindrique (5) étant montée à libre rotation dans le fourreau (6), l'élément fémoral (1) peut se déplacer en rotation au fur et à mesure de sa flexion correspondant au mouvement naturel de flexion de l'articulation du genou.

Le déplacement vertical de la tige (5) et par conséquent de l'ensemble de l'élément fémoral (1) par rapport à l'élément tibial (2), est limité par un jonc élastique (8) logé dans une gorge circulaire (6a) du fourreau (6). Le jonc (8) coopère en position d'accouplement de la tige (5) dans l'alésage du fourreau (6), avec un évidement (5a) sous forme d'une portée circulaire que présente ladite tige (5). L'extrémité libre de la tige (5) présente une portée tronconique. Le jonc élastique (8) coopère en pression avec l'ensemble de la tige (5) en étant ainsi conformé pour être sollicité élastiquement en expansion à l'intérieur de la gorge (6a). Le jonc (8) exerce donc sur la tige une force de pression constante.

A noter d'une manière importante, qu'en position d'engagement du jonc (8) dans l'évidement circulaire (5a), l'axe vertical de la section transversale dudit jonc est décalée par rapport au bord externe correspondant de la tige, de sorte que le diamètre primitif du jonc est très légèrement supérieur au diamètre externe de la tige.

Au moment de l'introduction de la tige (5) dans le fourreau (6), [a portée tronconique permet son introduction sous un faible effort de poussée en écartant le jonc (8) à l'intérieur de la gorge (6a). Le jonc (8) toujours soumis à la force de pression vient se positionner après descente de la tige (5) dans le fourreau (6), dans l'empreinte circulaire (5a), correspondant à la position de verrouillage.

En fonction de la hauteur de l'évidement circulaire (5a), la tige (5) a donc la possibilité d'un déplacement en hauteur limité à l'interieur du fourreau (6).

Comme indiqué précédemment, dans cette position de verrouillage, c'est-à-dire lorsque le jonc (8) coopère avec la portée circulaire (5a), apparait le décalage. Pour assurer le désaccouplement, l'effort de traction est beaucoup plus important, étant donné que la partie de raccordement entre l'évidement circulaire (5a) et le corps de la tige doit vaincre la résistance résultant de la force de pression du jonc élastique (8). Pour se dégager, la partie de raccordement agit sur le jonc (8), à la façon d'une rampe compte-tenu du décalage.

A noter que pour augmenter l'effort de traction, on peut, soit augmenter l'intensité de la force, soit diminuer la valeur du décalage, soit cumuler ces deux paramètres.

Ces dispositions permettent donc :
- d'assurer l'introduction de la tige (1) dans le fourreau (6) selon une force d'intensité réduite et d'assurer le blocage en position avec, si nécessaire, la possibilité de rotation et de déplacement limité en hauteur,
- d'assurer le retrait de la tige du fourreau selon une force d'intensité supérieure, mais nécessaire à assurer le désaccouplement.

Par ailleurs, compte-tenu des dispositions constructives de la prothèse, notamment au niveau de l'accouplement des éléments fémoral (1) et tibial (2), des résultats techniques avantageux peuvent être obtenus.

En position d'extension, la rotation dans le plan horizontal n'est pas possible en considérant l'emboitement de la forme trapézoïdale arrière mâle (6b) du fourreau (6) avec l'échancrure intercondylienne de l'élément fémoral (1) (figure 5).

En position de flexion, la rotation angulaire de l'élément fémoral s'obtient dès le début de la flexion, de façon contrôlée, par le contact des faces internes de l'échancrure intercondylienne de l'élément (1) avec les faces pentées (6c) ou (6d) du fourreau (6) (figure 5). En outre, la flexion de l'élément fémoral (1) étant limitée par le bec (4a) du manchon (4) (tracé traits interrompus figure 4), on obtient une limitation angulaire de sécurité en flexion par butée positive dudit élément (1) sur le bec (4a).

Suivant une autre caractéristique importante de l'invention, les patins condyliens (1c) (1d) de l'élément fémoral (1), sont en appui sur un plateau tibial (9). Ce plateau (9) est monté sur l'embase tibiale (2b) avec capacité de déplacement angulaire limité dans un plan horizontal. A cet effet, le plateau (9) présente au niveau de son bord antérieur, des lumières profilées (9a) coopérant avec des pions (10) qui apparaissent en débordement de l'embase tibiale (2b). Plus particulièrement, ces pions (10) présente une tête (10a) coopérant avec les lumières (9a) de section transversale en T notamment.

Il apparait donc que selon ces dispositions, le mouvement angulaire de rotation entre l'élément tibial et fémoral, s'effectue entre le plateau tibial (9) et l'embase tibiale (2b), générant ainsi une importante surface de frottement dans le plan horizontal.

A noter également que les patins condyliens (1c) et (1d) ont un profit déterminé dans le plan frontal et sagittal, pour avoir un maximum de surface en contact pendant la flexion.

On prévoit d'interposer entre le manchon (5) et les joues de la chape (1b) de l'élément fémoral, des flasques en polyéthylène (non représentés).

Les avantages ressortent bien de la description.

## Revendications

1. Prothèse totale du genou comprenant un élément fémoral (1) recevant un axe transversal d'articulation (3) monté à libre rotation par rapport à une tige cylindrique (5) engagée à libre rotation dans un fourreau vertical (6) d'un élément tibial (2) pour permettre des mouvements de flexion et de rotation, ledit élément fémoral étant en appui par ses patins condyliens (1c) (1d) sur une embase tibiale (2b) de l'élément (2), par l'intermédiaire d'un plateau en polyéthylène (9) monté avec capacité de déplacement angulaire limité dans un plan horizontal sur ladite embase tibiale, les patins condyliens (1c,1d) étant seuls aptes à provoquer le déplacement angulaire du plateau en polyéthylène (9) sur l'embase tibiale (2b).

2. Prothèse selon la revendication 1, caractérisée en ce que l'axe d'articulation (3) est monté libre en rotation dans un manchon transversal (4) solidaire du fourreau vertical (6) de l'élément tibial, ledit manchon étant engagé dans une saillie creuse (1b) de l'élément fémoral, constituant une chape.

3. Prothèse selon la revendication 1, caractérisée en ce que l'axe d'articulation (3) est monté dans le manchon (4) au moyen d'organes de roulement (7) du type à aiguille.

4. Prothèse selon la revendication 2, caractérisée en ce que le manchon (4) présente très sensiblement au niveau du plateau tibial, un bec (4a) faisant office de butée, coopérant avec une partie de la saillie (1b), en position de flexion de l'élément fémoral.

5. Prothèse selon la revendication 1, caractérisée en ce que le plateau (9) est solidaire de l'élément tibial avec capacité de déplacement angulaire, au moyen de pions (10) solidaires dudit élément et coopérant par une tête profilée (10a) avec des lumières profilées (9a), formées dans l'épaisseur dudit plateau.

6. Prothèse selon la revendication 1, caractérisée en ce que le fourreau (6) présente au moins un moyen (8) apte à coopérer en pression avec la tige (5), ledit moyen (8) étant conformé pour être sollicité élastiquement en combinaison avec des agencements de la tige (5) et du fourreau (6) pour permettre :
- l'introduction de la tige dans le fourreau selon une force d'intensité réduite et le blocage en position,
- le retrait de la tige du fourreau selon une force d'intensité supérieure à la précédente correspondant au désaccouplement.

7. Prothèse selon la revendication 6, caractérisée en ce que les agencements sont constitués par une portée conique formée en bout de la tige et par une gorge circulaire (6a) formé dans le fourreau (6) en étant apte à recevoir le ou les moyens (8) selon une position déterminée par rapport à une partie de la tige recevant le ou lesdits moyens (8) en position de blocage.

8. Prothèse selon la revendication 1, caractérisée en ce que la partie de la tige (5) recevant le ou les moyens (3) en position de blocage, est constituée par un évidemment (5a) dans lequel sont engagés par élasticité le ou les moyens (8), après introduction de la tige (5) dans le fourreau (6).

9. Prothèse selon la revendication 6, caractérisée en ce que le moyen de blocage (8) est réalisé sous forme d'un joint élastique.

10. Prothèse selon la revendication 9, caractérisée en ce qu'en position de coopération du jonc (8) avec l'évidement (5a), son diamètre moyen est supérieur ou au moins égal au diamètre externe de la tige.

## Claims

1. Complete knee prosthesis comprising a femoral element (1) accommodating a transverse hinge pin (3) mounted so that it can rotate freely relative to a cylindrical rod (5) fitted so that it can rotate freely in a vertical sleeve (6) of a tibial element (2) in order to allow flexing and rotating movements, the condylar pads (1c) (1d) of said femoral element resting on a tibial base (2b) of element (2) through a polyethylene plate (9) mounted so that it is capable of limited angular displacement in a horizontal plane on said tibial base, the condylar pads (1c, 1d) alone being capable of causing angular displacement of polyethylene plate (9) on tibial base (2b).

2. Prosthesis as claimed in claim 1 characterised in that hinge pin (3) is mounted so that it can rotate freely in a transverse bush (4) joined to vertical sleeve (6) of the tibial element, said bush being fitted into a hollow protrusion (1b) of the femoral element constituting a fork joint.

3. Prosthesis as claimed in claim 1 characterised in that hinge pin (3) is mounted in bush (4) by means of needle-type roller bearings (7).

4. Prosthesis as claimed in claim 2 characterised in that bush (4) has, substantially at the level of the tibial plate, a spur (4a) that acts as a limit stop and co-operates with a part of the protrusion (1b) when the femoral element is in the flexed position.

5. Prosthesis as claimed in claim 1 characterised in that plate (9) is joined to the tibial element with the capacity for angular displacement by means of studs (10) joined to said element and co-operating, through a shaped head (10a), with shaped slots (9a) formed in the thickness of said plate.

6. Prosthesis as claimed in claim 1 characterised in that sleeve (6) has at least one means (8) capable of co-operating in compression with rod (5), said means (8) being devised to be elastically stressed in combination with features of rod (5) and of sleeve (6) in order to allow:
- insertion of the rod into the sleeve by exerting a slight force and locking in position,
- withdrawal of the rod from the sleeve, by exerting a force in excess of the above force, which corresponds to releasing it.

7. Prosthesis as claimed in claim 6 characterised in that the features consist of a tapered bearing surface formed at the end of the rod and of a circular throat (6a) formed in sleeve (6), said throat being capable of accommodating means (8) in a predetermined position relative to a part of the rod that accommodates said means (8) in the locked position.

8. Prosthesis as claimed in claim 1 characterised in that the part of rod (5) that accommodates means (3) in the locked position consists of a recess (5a) in which means (8) is/are fitted by elasticity after inserting rod (5) into sleeve (6).

9. Prosthesis as claimed in claim 6 characterised in that means of locking (8) is produced in the form of an elastic ring.

10. Prosthesis as claimed in claim 9 characterised in that, in the position where ring (8) co-operates with recess (5a), its average diameter is equal to or greater than the outside diameter of the rod.

## Patentansprüche

1. Knie-Vollprothese, bestehend aus einem Femoralteil (1) zur Aufnahme einer querverlaufenden Schwenkachse (3), die gegenüber einem freidrehend in einem senkrechten Futteral (6) eines Tibialteils (2) sitzenden zylindrischen Stift (5) angeordnet ist, um Beuge- und Drehbewegungen zu ermöglichen, während das Femoralteil mit seinen Gelenkkapseln (1c) (1d) über eine Zwischenplatte (9) aus Polyethylen auf einem Tibialsockel (2b) des Tibialteils (2) aufliegt, wobei die Zwischenplatte (9) in einem begrenzten Winkelbereich auf der waagrechten Ebene des Tibialsockels schwenkbar ist und nur die Gelenkkapseln (1c, 1d) die Schwenkung der Zwischenplatte (9) aus Polyethylen auf dem Tibialsockel (2b) bewirken können.

2. Prothese nach Anspruch 1, dadurch gekennzeichnet, daß die Schwenkachse (3) freidrehend in einer fest mit dem senkrechten Futteral (6) des Tibialteils (2) verbundenen, querverlaufenden Muffe (4) angeordnet ist, welche in einem ausgehöhlten Vorsprung (1b) des Femoralteils (1) sitzt, wodurch ein Gabelgelenk gebildet wird.

3. Prothese nach Anspruch 1, dadurch gekennzeichnet, daß die Schwenkachse (3) unter Verwendung von Wälzlagerteilen (7) von der Art der Nadellager in der Muffe (4) angeordnet ist.

4. Prothese nach Anspruch 2, dadurch gekennzeichnet, daß die Muffe (4) weitgehend in Höhe der Zwischenplatte eine als Anschlag dienende Nase (4a) aufweist, die in der Beugestellung des Femoralteils mit einem Teil des Vorsprungs (1b) zusammenwirkt.

5. Prothese nach Anspruch 1, dadurch gekennzeichnet, daß die Zwischenplatte (9) fest mit dem Tibialteil verbunden ist und dabei in einem bestimmten Winkelbereich anhand von Nippeln (10) schwenkbar ist, welche fest mit dem Tibialteil verbunden sind und über einen Profilkopf (10a) mit im Material der Zwischenplatte eingearbeiteten Profilaussparungen (9a) zusammenwirken.

6. Prothese nach Anspruch 1, dadurch gekennzeichnet, daß das Futteral (6) mindestens ein Mittel (8) aufweist, das unter Druck mit dem Stift (5) zusammenwirken kann und so geformt ist, daß es in Verbindung mit bestimmten Teilen am Stift (5) und am Futteral (6) elastisch beansprucht wird, um
- den Stift mit geringem Kraftaufwand in das Futteral einführen und in Endstellung blockieren zu können,
- den Stift unter höherem Kraftaufwand als dem zum Einführen erforderlichen aus dem Futteral herausziehen zu können, um die beiden Teile zu trennen.

7. Prothese nach Anspruch 6, dadurch gekennzeichnet, daß die bestimmten Teile aus einer am Ende des Stiftes ausgebildeten konischen Lagerfläche sowie einer im Futteral (6) ausgebildeten ringförmigen Rille (6a) bestehen, die das bzw. die Mittel (8) entsprechend einer bestimmten Lage in Bezug auf einen Teil des Stiftes aufnehmen kann, der das bzw. die Mittel (8) in Blockierstellung aufnimmt.

8. Prothese nach Anspruch 1, dadurch gekennzeichnet, daß der Teil des Stiftes (5), der das bzw. die Mittel (8) in Blockierstellung aufnimmt, aus einer Vertiefung (5a) gebildet ist, in die sich das bzw. die Mittel (8) nach dem Einführen des Stiftes (5) in das Futteral (6) elastisch einfügen.

9. Prothese nach Anspruch 6, dadurch gekennzeichnet, daß das Blockiermittel (8) aus einem elastischen Ring besteht.

10. Prothese nach Anspruch 9, dadurch gekennzeichnet, daß der Ring (8) beim Zusammenwirken mit der Aushöhlung (5a) einen mittleren Durchmesser aufweist, dessen Wert größer oder gleich dem Wert des Außendurchmessers des Stiftes ist.
